# EUROPEAN PATENT SPECIFICATION

(11) **EP 2 808 424 B1**
(45) Date of publication and mention of the grant of the patent: **02.05.2018**
(21) Application number: 13740580.9
(22) Date of filing: 23.01.2013
(51) Int. Cl.: C25B 3/04, C25B 11/08, B01J 19/24

(54) **ELECTROCHEMICAL REDUCTION DEVICE AND METHOD FOR PRODUCING HYDRIDE OF NITROGEN-CONTAINING-HETEROCYCLIC AROMATIC COMPOUND OR AROMATIC HYDROCARBON COMPOUND**
VORRICHTUNG FÜR ELEKTROCHEMISCHE REDUKTION UND VERFAHREN ZUR HYDRIERUNG EINER STICKSTOFF ENTHALTENDEN HETEROCYCLISCHEN AROMATISCHEN VERBINDUNG ODER EINER AROMATISCHEN KOHLENWASSERSTOFFVERBINDUNG
DISPOSITIF DE RÉDUCTION ÉLECTROCHIMIQUE ET PROCÉDÉ DE PRODUCTION D'UN HYDRURE D'UN COMPOSÉ AROMATIQUE HÉTÉROCYCLIQUE CONTENANT DE L'AZOTE OU UN COMPOSÉ D'HYDROCARBURE AROMATIQUE

(30) Priority: 24.01.2012 JP 2012012355
(43) Date of publication of application: 03.12.2014
(73) Proprietor: JX Nippon Oil & Energy Corporation, Chiyoda-ku Tokyo 100-8162 (JP)
(72) Inventor: SATO, Yasushi, Tokyo 100-8162 (JP); MIYOSHI, Kota, Tokyo 100-8162 (JP); NAKAGAWA, Kojiro, Tokyo 100-8162 (JP); KOBORI, Yoshihiro, Tokyo 100-8162 (JP)
(74) Representative: Algemeen Octrooi- en Merkenbureau B.V.
(86) International application number: PCT/JP2013/000330
(87) International publication number: WO 2013/111585

(56) References cited:
- WO-A1-2011/122155
- JP-A- 2003 045 449
- US-A1- 2004 161 641
- US-A1- 2009 000 574

## Description

### [TECHNICAL FIELD]

The present invention relates to a device and a method for electrochemically hydrogenating an aromatic hydrocarbon compound or an N-containing heterocyclic aromatic compound.

### [BACKGROUND ART]

It is known that cyclic organic compounds such as cyclohexane and decalin are able to be obtained efficiently by hydrogenating a benzene ring of corresponding aromatic hydrocarbon compounds (benzene and naphthalene) using a hydrogen gas. Since high temperature and high pressure are required for this reaction, this reaction is not suitable for small and medium-scale manufacturing. On the other hand, in the case of an electrochemical reaction where an electrolysis cell is used, it is not necessary to treat gaseous hydrogen since water can be used as a source of hydrogen, and the reaction is known to progress under relatively mild reaction conditions (at about room temperature to 200°C and under normal pressure).

However, in order to reduce these compounds having low affinity with water and low electrical conductivity by a water-derived electrochemical reaction, it is obvious that ingenuity is required for an electrode
structure and electrode materials. Thus, it has been necessary to develop an electrode catalyst that is preferred for this purpose in order to perform a reduction reaction of an aromatic hydrocarbon compound with low consumption energy and high efficiency.

### [prior art document]

### [patent document]

[Patent document No. 1] JP 2003-045449
[Patent document No. 2] JP 2005-126288
[Patent Document No. 3] JP 2005-239479

### [non-patent document]

[non-patent document No. 1] Masaru Ichikawa, J. Jpn. Inst. Energy, vol. 85, 517 (2006)

### [DISCLOSURE OF THE INVENTION]

### [PROBLEM TO BE SOLVED BY THE INVENTION]

As an example for electrochemically hydrogenating an aromatic hydrocarbon compound such as toluene or the like, a method has been reported for obtaining methylcyclohexane, which is a hydride in which a benzene ring is hydrogenated, without going through a state of a hydrogen gas by sending toluene that is vaporized into a gaseous state to the side of a reduction electrode in a configuration similar to that of water electrolysis (see non-patent document No. 1). However, the amount of substance (current density) that can be transformed per electrode area and time is not large, and there is room for improving an electrode structure, in particular, an electrode on the reduction side in order to increase the amount of substance that can be processed per device volume.

In electrochemically reducing aromatic hydrocarbon compounds, both the supply of protons, which are transported from a counter electrode (oxygen evolution electrode), electrons, and aromatic hydrocarbon compounds serving as raw materials, and the discharge of generated hydrides in which a benzene ring is hydrogenated need to be carried out at the same time at a reduction electrode. In order to efficiently form a reactive interface during the transportation of these protons, electrons, and substances so as to obtain, for example, cyclohexane by six-electron reduction in the case of benzene and decalin by ten-electron reduction in the case of naphthalene, high electrocatalytic activity is required, and a three-dimensional structure for the formation of a reactive interface is also important.

In this background, a purpose of the present invention is to provide a technology capable of electrochemically hydrogenating at least one benzene ring of an aromatic hydrocarbon compound or an N-containing heterocyclic aromatic compound with high efficiency.

### [MEANS TO SOLVE THE PROBLEM]

One embodiment of the present invention relates to an electrochemical reduction device. The electrochemical reduction device comprises: an electrolyte membrane that has protonic conductivity; a reduction electrode that is provided on one side of the electrolyte membrane and that contains a reduction catalyst for hydrogenating at least one benzene ring of an aromatic hydrocarbon compound; and an oxygen evolving electrode that is provided on the other side of the electrolyte membrane, wherein the reduction catalyst is a composition that includes a first catalyst metal containing at least one of Pt and Pd and includes one or more kinds of second catalyst metals selected from among Cr, Mn, Fe, Co, Ni, Cu, Zn, Mo, Ru, Sn, W, Re, Pb, and Bi. In the hydrogenation of a benzene ring of an aromatic hydrocarbon compound, the number of benzene rings that are hydrogenated is not limited to one and may be two or more.

The ratio of the first catalyst metal to the total mass of the first catalyst metal and the second catalyst metal is between 10 wt% and 95 wt%, both inclusive. The first catalyst metal and the second catalyst metal are supported on a conductive material containing any one of a porous carbon, a porous metal, and a porous metal oxide. The conductive material has an electrical conductivity of 1.0*10⁻² S/cm or greater. The reduction electrode may contain a protonic conductor. The protonic conductor may be an ion-conductive polymer. The catalyst metal may be partially coated by the protonic conductor.

Another embodiment of the present invention relates to a method for manufacturing a hydride of an aromatic hydrocarbon compound or an N-containing heterocyclic aromatic compound. In the method for manufacturing a hydride of an aromatic hydrocarbon compound or an N-containing heterocyclic aromatic compound, by introducing an aromatic hydrocarbon compound or an N-containing heterocyclic aromatic compound to the reduction electrode side of the electrochemical reduction device according to any one of the above-stated embodiments while circulating water or a humidified gas to the oxygen evolving electrode side and then by externally applying an electric field such that the reduction electrode and the oxygen evolving electrode have a less-noble potential and a noble potential, respectively, at least one benzene ring of the aromatic hydrocarbon compound or the N-containing heterocyclic aromatic compound introduced to the reduction electrode side is hydrogenated.

In the method for manufacturing a hydride of an aromatic hydrocarbon compound or an N-containing heterocyclic aromatic compound, the aromatic hydrocarbon compound or the N-containing heterocyclic aromatic compound to be introduced to the reduction electrode side may be introduced in a liquid state at a reaction temperature.

Combinations of the above-stated elements will also be within the scope of the present invention sought to be patented by the present patent application.

### [ADVANTAGE OF THE INVENTION]

According to the present invention, at least one benzene ring of an aromatic hydrocarbon compound or an N-containing heterocyclic aromatic compound can be electrochemically hydrogenated with high efficiency.

### [BRIEF DESCRIPTION OF THE DRAWINGS]

Fig. 1 is a schematic diagram illustrating the configuration of an electrochemical reduction device according to an embodiment;
Fig. 2 is a diagram illustrating the configuration of an electrolysis cell of the electrochemical reduction device according to the embodiment;
Fig. 3 is a graph illustrating a relationship between a catalyst metal used for a reduction electrode and current density;
Fig. 4 is a graph illustrating a relationship between the content rate of a second catalyst metal and relative current density; and
Fig. 5 is a graph illustrating a relationship between an ionomer ratio and relative current density.

### [BEST MODE FOR CARRYING OUT THE INVENTION]

Described below is an explanation of the embodiments of the present invention with reference to figures. In the figures, like numerals represent like constituting elements, and the description thereof is omitted appropriately.

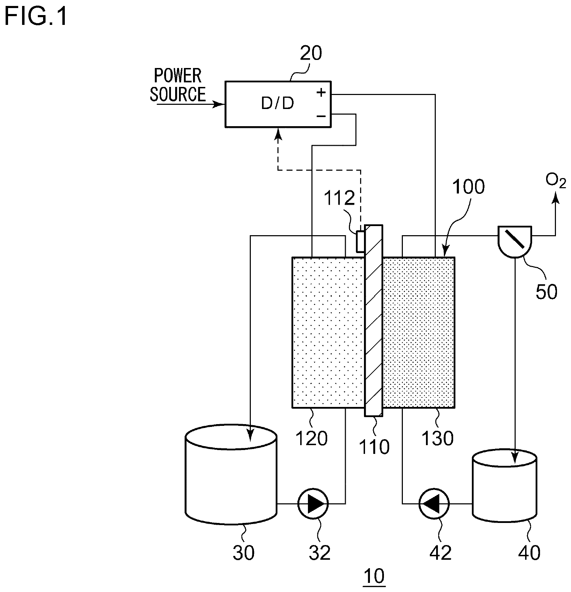

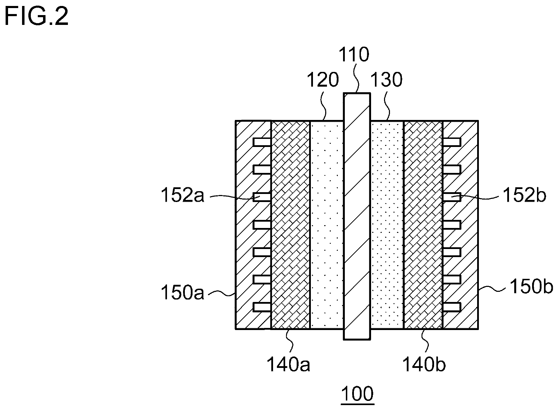

Fig. 1 is a schematic diagram illustrating the configuration of an electrochemical reduction device according to an embodiment. Fig. 2 is a diagram illustrating the configuration of an electrolysis cell of the electrochemical reduction device according to the embodiment. As shown in Fig. 1, an electrochemical reduction device 10 has an electrolysis cell 100, a power control unit 20, an organic material storage tank 30, a water storage tank 40, and a gas-liquid separation unit 50.

The power control unit 20 is, for example, a DC/DC converter for converting the output voltage of a power source into a predetermined voltage. The positive electrode output terminal of the power control unit 20 is connected to the positive electrode of the electrolysis cell 100. The negative electrode output terminal of the power control unit 20 is connected to the negative electrode of the electrolysis cell 100. With this, a predetermined voltage is applied between an oxygen evolving electrode (positive electrode) 130 of the electrolysis cell 100 and a reduction electrode (negative electrode) 120. A reference electrode input terminal of the power control unit 20 is connected to a reference electrode 112 provided on an electrolyte membrane 110, which will be described later, and the potential of the positive electrode output terminal and the potential of the negative electrode output terminal are determined based on the potential of the reference electrode 112. As the power source, electrical power derived from natural energy such as sunlight, wind power, and the like can be used.

The organic material storage tank 30 stores an aromatic compound. An aromatic compound used in the present embodiment is an aromatic hydrocarbon compound or an N-containing heterocyclic aromatic compound containing at least one aromatic ring and includes benzene, naphthalene, anthracene, 1,1-diphenylethane, 1,2-diphenylethane, pyridine, pyrimidine, pyrazine, quinoline, isoquinoline, N-alkylpyrrole, N-alkylindole, N-alkyldibenzopyrrole and the like. One through four hydrogen atoms of the aromatic ring(s) of the aromatic hydrocarbon compound or the N-containing heterocyclic aromatic compound may be substituted with alkyl groups. An "alkyl" of the above aromatic compounds is a linear or branched alkyl group with one through six carbons. For example, alkylbenzene includes toluene, ethyl benzene, and the like, dialkylbenzene includes o-xylene, m-xylene, p-xylene, o-diethylbenzene, m-diethylbenzene, p-diethylbenzene, and the like, and trialkylbenzene includes mesitylene and the like. An example of alkylnaphthalene includes methylnaphthalene. The above-stated aromatic ring(s) of the aromatic hydrocarbon compound or the N-containing heterocyclic aromatic compound may have one through three substituents. In the following explanation, an aromatic hydrocarbon compound and an N-containing heterocyclic aromatic compound used in the present invention are each often referred to as an "aromatic compound". Preferably, an aromatic compound is a liquid at room temperature. Since a plurality of aromatic compounds can be used as a mixture, it is only necessary for the mixture to show a stable liquid state. With this, the aromatic compound can be supplied to the electrolysis cell 100 in a liquid state without performing processes such as heating, pressurizing, and the like. Thus, the simplification of the electrochemical reduction device 10 can be achieved. The concentration of the aromatic compound in a liquid state is 0.1 percent or greater, preferably 0.3 percent or greater, and more preferably 0.5 percent or greater.

The aromatic compound stored in the organic material storage tank 30 is supplied to the reduction electrode 120 of the electrolysis cell 100 by a first liquid supply device 32. For the first liquid supply device 32, for example, various types of pumps such as a gear pump, a cylinder pump, or the like or a gravity flow device or the like can be used. A circulation pathway is provided between the organic material storage tank 30 and the reduction electrode of the electrolysis cell 100. An aromatic compound in which at least one benzene ring is hydrogenated by the electrolysis cell 100 and an unreacted aromatic compound are stored in the organic material storage tank 30 via the circulation pathway. No gas is generated by a major reaction that progresses at the reduction electrode 120 of the electrolysis cell 100. In the case where hydrogen is produced as a byproduct, a gas-liquid separation device may be provided in the middle of the circulation pathway.

The water storage tank 40 stores ion-exchanged water, purified water, and the like (hereinafter, simply referred to as "water"). Water stored in the water storage tank 40 is supplied to the oxygen evolving electrode 130 of the electrolysis cell 100 by a second liquid supply device 42. As in the case of the first liquid supply device 32, for example, various types of pumps such as a gear pump, a cylinder pump, or the like or a gravity flow device or the like can be used for the second liquid supply device 42. A circulation pathway is provided between the water storage tank 40 and the oxygen evolving electrode of the electrolysis cell 100. Water that is unreacted in the electrolysis cell 100 is stored in the water storage tank 40 via the circulation pathway. The gas-liquid separation unit 50 is provided in the middle of a pathway where unreacted water is sent back to the water storage tank 40 from the electrolysis cell 100. The gas-liquid separation unit 50 separates oxygen produced by the electrolysis of water in the electrolysis cell 100 from water and discharges the oxygen outside the system.

As shown in Fig. 2, the electrolysis cell 100 has an electrolyte membrane 110, a reduction electrode 120, an oxygen evolving electrode 130, liquid diffusion layers 140a and 140b, and separators 150a and 150b.

The electrolyte membrane 110 is formed of a material (ionomer) having protonic conductivity. While selectively conducting protons, the electrolyte membrane 110 is required to prevent substances from getting mixed or being diffused between the reduction electrode 120 and the oxygen evolving electrode 130. The thickness of the electrolyte membrane 110 is preferably from 5 to 300 µm, more preferably from 10 to 150 µm, and most preferably from 20 to 100 µm. If the thickness of the electrolyte membrane 110 is less than 5 µm, the barrier property of the electrolyte membrane 110 is lowered, and the amount of cross-leaking substances is more likely to increase. If the thickness of the electrolyte membrane 110 is more than 300 µm, ion transfer resistance becomes too large. Thus, the thickness of more than 300 µm is not preferred.

The sheet resistance, i.e., ion transfer resistance per geometric area, of the electrolyte membrane 110 is preferably 2000 mΩ·cm² or less, more preferably 1000 mΩ·cm² or less, and most preferably 500 mΩ·cm² or less. If the contact resistance of the electrolyte membrane 110 is 2000 mΩ·cm² or greater, protonic conductivity becomes insufficient. An example of a material having protonic conductivity (which is a cation-exchanging ionomer) includes a perfluorosulfonic acid polymer such as Nafion (registered trademark), Flemion (registered trademark), etc. The ion exchange capacity (IEC) of the cation-exchanging ionomer is preferably from 0.7 to 2 meq/g and more preferably from 1 to 1.2 meq/g. If the ion exchange capacity of the cation-exchanging ionomer is less than 0.7 meq/g, ionic conductivity becomes insufficient. On the other hand, if the ion exchange capacity of the cation-exchanging ionomer is greater than 2 meq/g, the solubility of the ionomer in water becomes increased, and the strength of the electrolyte membrane 110 thus becomes insufficient.

The reduction electrode 120 is provided on one side of the electrolyte membrane 110. The reduction electrode 120 is a reduction electrode catalyst layer containing a reduction catalyst for hydrogenating at least one benzene ring of an aromatic compound. A reduction catalyst used for the reduction electrode 120 is composed of a composition containing a first catalyst metal (noble metal) that contains at least one of Pt and Pd and one or more kinds of second catalyst metals selected from among Cr, Mn, Fe, Co, Ni, Cu, Zn, Mo, Ru, Sn, W, Re, Pb, and Bi. The form of the first catalyst metal and the form of the second catalyst metal included in the composition are alloys of the first catalyst metal and the second catalyst metal or intermetallic compounds composed of the first catalyst metal and the second catalyst metal. The ratio of the first catalyst metal to the total mass of the first catalyst metal and the second catalyst metal is preferably from 10 to 95 wt%, and more preferably from 20 to 90 wt%, and most preferably from 25 to 80 wt%. The ratio of the first catalyst metal of less than 10 wt% may result in deterioration in durability from the perspective of resistance to dissolving or the like. On the other hand, if the ratio of the first catalyst metal is greater than 95 wt%, the properties of the reduction catalyst become similar to the properties of a noble metal alone, and the electrode activity thus becomes insufficient. Depending on a combination of a first catalyst metal and a second catalyst metal that are selected, the rate of alloying or the rate of forming an intermetallic compound in the composition is different. Therefore, by adjusting the mass of one or more kinds of second catalyst metals such that the content ratio of the first catalyst metal falls within the range of the above values, either performance or economic advantages or the both can be achieved. In the following explanation, a first catalyst metal and a second catalyst metal are often collectively referred to as "catalyst metals".

The above-described catalyst metals may be supported by a conductive material (support). The electrical conductivity of the conductive material is preferably 1.0*10⁻² S/cm or greater, more preferably 3.0*10⁻² S/cm or greater, and most preferably 1.0*10⁻¹ S/cm or greater. If the electrical conductivity of the conductive material is less than 1.0*10⁻² S/cm, sufficient conductivity cannot be provided. An example of the conductive material includes a conductive material containing any one of a porous carbon, a porous metal, and a porous metal oxide as a major component. An example of the porous carbon includes carbon black such as ketjen black (registered trademark), acetylene black, vulcan (registered trademark), or the like. The BET specific surface area of the porous carbon measured by a nitrogen adsorption method is preferably 100 m²/g or greater, more preferably 150 m²/g or greater, and most preferably 200 m²/g or greater. If the BET specific surface area of the porous carbon is less than 100 m²/g, it is difficult to uniformly support the catalyst metals. Therefore, the rate of utilization of a catalyst metal surface is lowered, causing the catalyst performance to be lowered. Examples of the porous metal include, for example, Pt black, Pd black, a Pt metal deposited in a fractal shape, and the like. Examples of a porous metal oxide include an oxide of Ti, an oxide of Zr, an oxide of Nb, an oxide of Mo, an oxide of Hf, an oxide of Ta, and an oxide of W. Furthermore, examples of a porous conductive material for supporting a catalyst metal include a nitride, a carbide, an oxynitride, a carbonitride, a partially-oxidized carbonitride of a metal such as Ti, Zr, Nb, Mo, Hf, Ta, W, or the like (hereinafter, these are collectively referred to as porous metal carbonitrides and the like). The respective BET specific surface areas of the porous metal, the porous metal oxide, the porous metal carbonitrides, and the like measured by a nitrogen adsorption method are preferably 1 m²/g or greater, more preferably 3 m²/g or greater, and most preferably 10 m²/g or greater. If the respective BET specific surface areas of the porous metal, the porous metal oxide, the porous metal carbonitrides, and the like are less than 1 m²/g, it is difficult to uniformly support the catalyst metals. Therefore, the rate of utilization of a catalyst metal surface is lowered, causing the catalyst performance to be lowered.

The ratio of a composition composed of the first catalyst metal and the second catalyst metal to the total mass of the first catalyst metal, the second catalyst metal, and the support, in other words, a support rate, is preferably from 1 to 90 wt%, and more preferably from 3 to 75 wt%, and most preferably from 5 to 50 wt%. If the support rate is less than 1 wt%, the activity per area in the reduction electrode 120 becomes insufficient. On the other hand, if the support rate is greater than 90 wt%, it becomes difficult to highly disperse the catalyst metals, and aggregation becomes more likely to be caused. Since the amount of the noble metal will be increased, the manufacturing costs of the reduction electrode 120 will be increased.

Depending on the type and composition of the first catalyst metal and the second catalyst metal, a simultaneous impregnation method or a sequential impregnation method can be employed as a method for supporting the catalyst metals on the support. The first catalyst metal and the second catalyst metal are simultaneously impregnated into the support in the simultaneous impregnation method, and the second catalyst metal is impregnated into the support after the first catalyst metal is impregnated into the support in the sequential impregnation method. In the case of the sequential impregnation method, after the first catalyst metal is loaded onto the support, a heat treatment or the like may be performed once, and the second catalyst metal may be then loaded onto the support. After the impregnation of both the first catalyst metal and the second catalyst metal is completed, the alloying of the first catalyst metal and the second catalyst metal or the formation of an intermetallic compound composed of the first catalyst metal and the second catalyst metal is performed by a heat treatment process.

A material having conductivity such as the previously-stated conductive oxide, carbon black, or the like may be added to the reduction electrode 120 in addition to a conductive compound on which a catalyst metal is supported. With this, the number of electron-conducting paths among reduction catalyst particles can be increased. Thus, resistance per geometric area of a reduction catalyst layer can be lowered in some cases.

As an additive agent, a fluorine-based resin such as polytetrafluoroethylene (PTFE) may be contained in the reduction electrode 120.

The reduction electrode 120 may contain an ionomer having protonic conductivity. Preferably, the reduction electrode 120 contains ionically conducting materials (ionomers) having a structure that is identical or similar to that of the above-stated electrolyte membrane 110 in a predetermined mass ratio. This allows the ionic conductivity of the reduction electrode 120 to be improved. In particular, in the case where a catalyst support is porous, the reduction electrode 120 makes a significant contribution to the improvement of the ionic conductivity by containing an ionomer that has protonic conductivity. An example of an ionomer having protonic conductivity (which is a cation-exchanging ionomer) includes a perfluorosulfonic acid polymer such as Nafion (registered trademark), Flemion (registered trademark), etc. The ion exchange capacity (IEC) of the cation-exchanging ionomer is preferably from 0.7 to 3 meq/g, more preferably from 1 to 2.5 meq/g, and most preferably from 1.2 to 2 meq/g. When a catalyst metal is supported on porous carbon (carbon support), a mass ratio I/C of the cation-exchanging ionomer (I) to the carbon support (C) is preferably from 0.1 to 2, more preferably from 0.2 to 1.5, and most preferably from 0.3 to 1.1. It is difficult to obtain sufficient ionic conductivity if the mass ratio I/C is less than 0.1. On the other hand, if the mass ratio I/C is 2 or greater, an increase in the thickness of an ionomer coating for the catalyst metal inhibits an aromatic compound, which is a reactant, from touching a catalytic site, and a decrease in the electron conductivity lowers the electrode activity.

Preferably, the ionomers contained in the reduction electrode 120 partially coat a reduction catalyst. This allows three elements (an aromatic compound, a proton, and an electron) that are necessary for an electrochemical reaction at the reduction electrode 120 to be efficiently supplied to a reaction field.

The liquid diffusion layer 140a is laminated on the surface of the reduction electrode 120 on the side opposite to the electrolyte membrane 110. The liquid diffusion layer 140a plays a function of uniformly diffusing, to the reduction electrode 120, a liquid aromatic compound supplied from the separator 150a that is described later. As the liquid diffusion layer 140a, for example, carbon paper and carbon cloth are used.

The separator 150a is laminated on the surface of the liquid diffusion layer 140a on the side opposite to the electrolyte membrane 110. The separator 150a is formed of a carbon resin, an anticorrosion alloy of Cr-Ni-Fe, Cr-Ni-Mo-Fe, Cr-Mo-Nb-Ni, Cr-Mo-Fe-W-Ni, or the like. A single or a plurality of groove-like flow channels 152a is/are provided on the surface of the separator 150a on the side of the liquid diffusion layer 140a. The liquid aromatic compound supplied from the organic material storage tank 30 circulates through the flow channel 152a. The liquid aromatic compound soaks into the liquid diffusion layer 140a from the flow channel 152a. The form of the flow channel 152a is not particularly limited. For example, a straight flow channel or a serpentine flow channel can be used. When a metal material is used for the separator 150a, the separator 150a may be a structure in which ball-like or pellet-like metal fine powder is sintered.

The oxygen evolving electrode 130 is provided on the other side of the electrolyte membrane 110. The oxygen evolving electrode 130 that contains catalysts of noble metal oxides such as RuO2, IrO2, and the like is preferably used. These catalysts may be supported in a dispersed manner or coated by a metal substrate such as a metal wire or mesh of metals such as Cr, Mn, Fe, Co, Ni, Cu, Zn, Nb, Mo, Ta, W, and the like or of alloys composed primarily of these metals. In particular, since IrO2 is high-priced, manufacturing costs can be lowered by performing thin film coating on a metal substrate when IrO2 is used as a catalyst.

The liquid diffusion layer 140b is laminated on the surface of the oxygen evolving electrode 130 on the side opposite to the electrolyte membrane 110. The liquid diffusion layer 140b plays a function of uniformly diffusing, to the oxygen evolving electrode 130, water supplied from the separator 150b that is described later. As the liquid diffusion layer 140b, for example, carbon paper and carbon cloth are used.

The separator 150b is laminated on the surface of the liquid diffusion layer 140b on the side opposite to the electrolyte membrane 110. The separator 150b is formed of an anticorrosion alloy of Cr-Ni-Fe, Cr-Ni-Mo-Fe, Cr-Mo-Nb-Ni, Cr-Mo-Fe-W-Ni, or the like or of a material in which the surfaces of these metals are coated by an oxide film. A single or a plurality of groove-like flow channels 152b is/are provided on the surface of the separator 150b on the side of the liquid diffusion layer 140b. The water supplied from the water storage tank 40 circulates through the flow channel 152b. The water soaks into the liquid diffusion layer 140b from the flow channel 152b. The form of the flow channel 152b is not particularly limited. For example, a straight flow channel or a serpentine flow channel can be used. When a metal material is used for the separator 150b, the separator 150b may be a structure in which ball-like or pellet-like metal fine powder is sintered.

In the present embodiment, liquid water is supplied to the oxygen evolving electrode 130. Alternatively, a humidified gas (e.g., air) may be used in place of liquid water. In this case, the dew-point temperature of the humidified gas is preferably from room temperature to 100°C and more preferably from 50 to 100°C.

When toluene is used as the aromatic compound, reactions that occur in the electrolysis cell 100 are as follows:

### <Electrode Reaction at Oxygen Evolving Electrode>

3H₂O → 1.5O₂ + 6H+ + 6e- : E₀ = 1.23 V

### <Electrode Reaction at Reduction Electrode>

toluene + 6H⁺ + 6e⁻ → methylcyclohexane : E₀ = 0.153 V (vs RHE)

In other words, the electrode reaction at the oxygen evolving electrode and the electrode reaction at the reduction electrode progress in parallel, and protons produced by electrolysis of water are supplied to the reduction electrode via the electrolyte membrane 110 by the electrode reaction at the oxygen evolving electrode and used for hydrogenation of at least one benzene ring of the aromatic compound in the electrode reaction at the reduction electrode.

More specifically, by setting an electrode potential at the reduction electrode 120 to be less than or equal to the standard oxidation-reduction potential E0 of the aromatic compound used as a raw material and by setting an electrode potential at the oxygen evolving electrode 130 to be greater than an oxygen evolution potential, respective electrochemical reactions are progressed at both electrodes. Therefore, a voltage of an external electric field that is necessary for progressing the reactions is a voltage obtained by adding, to this potential difference, an overvoltage required for the reactions, an overvoltage for mass transfer diffusion, and a resistance loss (ohmic loss) caused by the resistance of the electrolyte membrane 110. The voltage of the external electric field is preferably from 1.2 to 2.4 V, more preferably from 1.3 to 2.0 V, and most preferably from 1.35 to 1.6 V. Theoretically, if the voltage of the external electric field is below 1.2 V, an electrode reaction does not progress. Thus, it is difficult to industrially practice hydrogenation of at least one benzene ring of the aromatic compound. The voltage of the external electric field of greater than 2.4 V is not preferred from the aspect of energy efficiency since an excess amount of electric energy provided from the external electric field will become necessary. An excessive decrease in the potential at the reduction electrode 120 side progresses side reactions (e.g., hydrogen generation) other than the hydrogenation of at least one benzene ring of the aromatic compound. On the other hand, an excessive increase in the potential at the oxygen evolving electrode 130 side causes corrosion of a catalyst used for the oxygen evolving electrode 130 to be progressed easily.

Furthermore, the following reaction conditions are used favorably for the hydrogenation of at least one benzene ring of an aromatic compound using the electrochemical reduction device 10. The temperature of the electrolysis cell 100 is preferably from room temperature to 100°C and more preferably from 40 to 80°C. The temperature of the electrolysis cell 100 of below the room temperature is not preferred since there is a possibility that the progress of an electrolytic reaction is slowed down or an enormous amount of energy is required to remove heat generated as the reaction progresses. On the other hand, the temperature of the electrolysis cell 100 of above 100°C is not preferred for the electrochemical reduction device 10 in which reactions of the both electrodes are performed in a liquid phase since water will be boiled at the oxygen evolving electrode 130 and the vapor pressure of an organic substance will be increased at the reduction electrode 120. The potential of the reduction electrode 120 is preferably from -100 to 150 mV (vs RHE), more preferably from -50 to 100 mV (vs RHE), and most preferably from -25 to 50 mV (vs RHE). The potential of the reduction electrode 120 of below -10 mV (vs RHE) is not preferred since competition with a hydrogen generation reaction will occur while decreasing the selectivity of the reduction of an organic substance. On the other hand, if the potential of the reduction electrode 120 is higher than 150 mV (vs RHE), a reaction rate (current density) that is practically sufficient will not be obtained. Thus, the potential of the reduction electrode 120 that is higher than 150 mV is not preferred.

A hydride in which at least one benzene ring is hydrogenated that is obtained in the electrochemical reduction device 10 is basically a complete reductant of the aromatic compound that serves as a raw material. As described above, when toluene is used as the aromatic compound, the nuclear hydride that can be obtained is methylcyclohexane. Furthermore, when naphthalene is used as the aromatic compound, the hydride in which two benzene rings is hydrogenated that can be obtained is decalin.

If the above-stated first catalyst metal (Pt, Pd) is used alone as a catalyst metal of the reduction electrode 120, a certain degree of electrode activity is exhibited. However, since the first catalyst metal is specifically absorbed to an active site of an aromatic compound having a π-electron system, the electrode activity is inhibited. On the other hand, in the present embodiment, by using an alloy or intermetallic compound composed of the first catalyst metal and the second catalyst metal for the catalyst metal of the reduction electrode 120, the catalyst metal is inhibited from being specifically absorbed to an active site of the aromatic compound. With this, the electrode activity of the reduction electrode 120 can be improved, and the hydrogenation of at least one benzene ring of the aromatic compound can be efficiently performed eventually.

### (Evaluation on Current Density)

An evaluation on current density when a hydrogenation reaction of a benzene ring of toluene was performed was conducted using electrolysis cells according to exemplary embodiments 1-7 and comparative examples 1-4. Composition and components used for the electrolysis cells according to exemplary embodiments 1-7 and comparative examples 1-4 are shown in Table 1.

**[TABLE 1]**

| | REDUCTION ELECTRODE | | | | ELECTROLYTE MEMBRANE | | OXYGEN EVOLVING ELECTRODE | CURRENT DENSITY (mA/cm²) |
|---|---|---|---|---|---|---|---|---|
| | REDUCTION CATALYST | CONDUCTIVE MATERIAL | IONOMER | DEPTH(*µ* m) | ION CONDUCTOR | DEPTH(*µ* m) | METAL CATALYST | |
| COMPARATIVE EXAMPLE 1 | Pt | Ketjen black EC600JD | Nafion DE2020 (EW=1100), I/C=0.5 | 30 | Nafion 117CS (EW=1100) | 175 | IrO₂/Mo | 41 |
| EXEMPLARY EMBODIMENT 1 | Pt(93wt%), Co(7wt%) | Ketjen black EC600JD | Nafion DE2020 (EW=1100), I/C=0.5 | 30 | Nafion 117CS (EW=1100) | 175 | IrO₂/Mo | 47 |
| EXEMPLARY EMBODIMENT 2 | Pt(90wt%), Ni(10wt%) | Ketjen black EC600JD | Nafion DE2020 (EW=1100), I/C=0.5 | 30 | Nafion 117CS (EW=1100) | 175 | IrO₂/Mo | 52 |
| EXEMPLARY EMBODIMENT 3 | Pt(56wt%), Ru(44wt%) | Ketjen black EC600JD | Nafion DE2020 (EW=1100), I/C=0.5 | 30 | Nafion 117CS (EW=1100) | 175 | IrO₂/Mo | 83 |
| EXEMPLARY EMBODIMENT 4 | Pt(65wt%), Ru(30wt%), Ni(5wt%) | Ketjen black EC600JD | Nafion DE2020 (EW=1100), I/C=0.5 | 30 | Nafion 117CS (EW=1100) | 175 | IrO₂/Mo | 80 |
| EXEMPLARY EMBODIMENT 5 | Pt(80wt%), Pb(20wt%) | Ketjen black EC600JD | Nafion DE2020 (EW=1100), I/C=0.5 | 30 | Nafion 117CS (EW=1100) | 175 | IrO₂/Mo | 62 |
| COMPARATIVE EXAMPLE 2 | Pd | Ketjen black EC600JD | Nafion DE2020 (EW=1100), I/C=0.5 | 30 | Nafion 117CS (EW=1100) | 175 | IrO₂/Mo | 15 |
| EXEMPLARY EMBODIMENT 6 | Pd(90wt%), Co(10wt%) | Ketjen black EC600JD | Nafion DE2020 (EW=1100), I/C=0.5 | 30 | Nafion 117CS (EW=1100) | 175 | IrO₂/Mo | 23 |
| EXEMPLARY EMBODIMENT 7 | Pd(70wt%), Ru(30wt%) | Ketjen black EC600JD | Nafion DE2020 (EW=1100), I/C=0.5 | 30 | Nafion 117CS (EW=1100) | 175 | IrO₂/Mo | 48 |
| COMPARATIVE EXAMPLE 3 | Co | Ketjen black EC600JD | Nafion DE2020 (EW=1100), I/C=0.5 | 30 | Nafion 117CS (EW=1100) | 175 | IrO₂/Mo | 6 |
| COMPARATIVE EXAMPLE 4 | Pb | Ketjen black EC600JD | Nafion DE2020 (EW=1100), I/C=0.5 | 30 | Nafion 117CS (EW=1100) | 175 | IrO₂/Mo | 1 |

Conditions for the hydrogenation reaction of two benzene rings are as shown in the following.
total mass of reduction catalyst metals: 0.5 mg/cm² potential of reduction electrode: 0 mV (vs RHE)
ionomer ratio (mass ratio I/C): 1

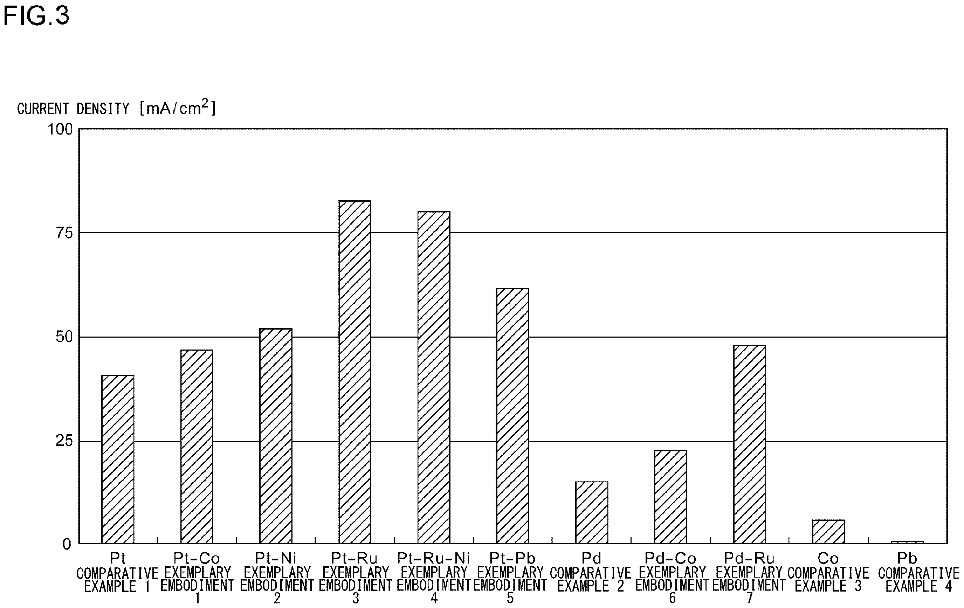

The current density flowing through each electrolysis cell is shown in Table 1. A relationship between each electrolysis cell and current density, i.e., a relationship between a catalyst metal used for a reduction electrode and current density is shown in Fig. 3.

As shown in Table 1 and Fig. 3, it has been verified that exemplary embodiments 1-5 that had a second catalyst metal (one or more kinds of catalyst metals selected from among Cr, Mn, Fe, Co, Ni, Cu, Zn, Mo, Ru, Sn, W, Re, Pb, and Bi) in addition to a first catalyst metal (Pt) showed current density that was higher than that of comparative example 1 that had only a first catalyst metal (Pt) as a catalyst metal. Also, it has been verified that exemplary embodiments 6-7 that had a second catalyst metal (one or more kinds of catalyst metals selected from among Cr, Mn, Fe, Co, Ni, Cu, Zn, Mo, Ru, Sn, W, Re, Pb, and Bi) in addition to a first catalyst metal (Pd) showed current density that was higher than that of comparative example 2 that had only a first catalyst metal (Pd) as a catalyst metal. It has been verified that comparative examples 3 and 4 that had only a second metal showed current density that was lower than that of comparative example 2.

### (Relationship between Current Density and Content Rate of Catalyst Metal)

Using as a reference a case where only a first catalyst metal was used, relative current density obtained when the content rate of a second catalyst metal was changed was evaluated. Table 2 shows combinations of first catalyst metals and second catalyst metals and relative current density obtained when the content rate of a second catalyst metal was changed. Since the evaluation was conducted using a composition composed of one kind of second catalyst metal along with one kind of first catalyst metal, Table 2 and Fig.

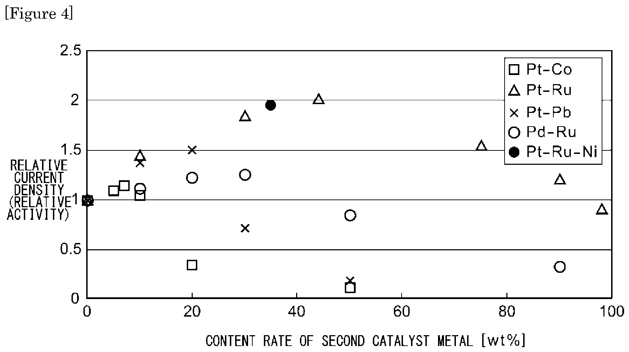

4 describe "Content Rate of Second Catalyst Metal (wt%)" for descriptive purposes. A value obtained by subtracting the content rate of the second catalyst metal from 100 corresponds to the content rate of the first catalyst metal. Fig. 4 is a graph illustrating a relationship between the content rate of a second catalyst metal and relative current density. The composition of an electrolysis cell is the same as that of the electrolysis cell used for the evaluation on current density except for components of a reduction catalyst metal.

**[TABLE 2]**

| CONTENT RATE OF SECOND CATALYST METAL (wt%) | RELATIVE CURRENT DENSITY | | | | |
|---|---|---|---|---|---|
| | Pt-Co | Pt-Ru | Pt-Ru-Ni | Pt-Pb | Pd-Ru |
| | | | (Ru30%,Ni5%) | | |
| 0 | 1 | 1 | 1 | 1 | 1 |
| 5 | 1.1 | - | - | - | - |
| 7 | 1.15 | - | - | - | - |
| 10 | 1.05 | 1.45 | - | 1.38 | 1.12 |
| 20 | 0.35 | - | - | 1.51 | 1.23 |
| 30 | - | 1.85 | - | 0.72 | 1.26 |
| 35 | - | - | 1.95 | - | - |
| 44 | - | 2.02 | - | - | - |
| 50 | 0.12 | - | - | 0.19 | 0.85 |
| 75 | - | 1.55 | - | - | - |
| 90 | - | 1.21 | - | - | 0.33 |
| 98 | - | 0.91 | - | - | - |

As shown in Table 2 and Fig. 4, it has been verified in all the combinations that relative current density gradually increased as the content rate of a second catalyst metal increased from zero and that relative current density gradually decreased once the content rate of a second catalyst metal exceeded a certain amount. While relative current density was 1.85 when the content rate of a second catalyst metal was 30 wt%, i.e., when the content rate of a first catalyst metal (Pt) was 70 wt%, relative current density was 1.95 when the content of a first catalyst metal was smaller than this having the content rate of a second catalyst metal of 35 wt%, i.e., when the content rate of the first catalyst metal (Pt) was 65 wt%. Thus, it has been verified that the same or higher current density was able to be obtained. In other words, by using two or more kinds of second catalyst metals, current density can be improved while suppressing the amount of the first catalyst metal (Pt) that is used.

### (Relationship between Current Density and Ionomer Ratio)

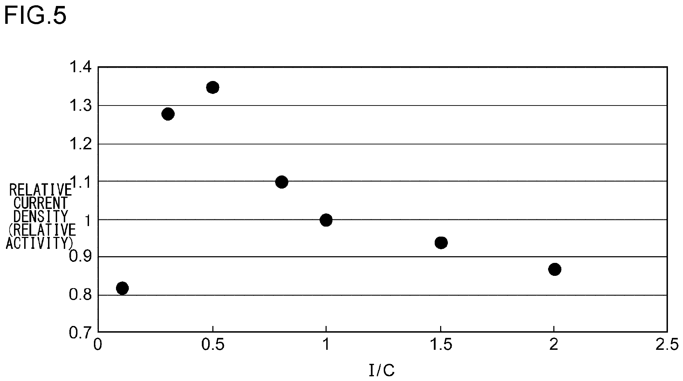

Using as a reference a case where an ionomer ratio was one, relative current density obtained when the ionomer ratio was changed was evaluated. The composition of an electrolysis cell used for the evaluation is the same as that of the electrolysis cell used for the evaluation on current density in Table 1. A relationship between an ionomer ratio (mass ratio I/C) and relative current density is shown in Table 3. Fig. 5 is a graph illustrating a relationship between an ionomer ratio and relative current density. As shown in Fig. 5, it has been verified that a good electrode activity was obtained when a mass ratio I/C was in a range from 0.1 to 2 and that an electrode activity was prominently improved when the mass ratio was in a range from 0.2 to 1.5 and in a range from 0.3 to 1.1 in said order. The evaluation was conducted using metal catalysts according to exemplary embodiment 3 described in Table 1. It can be said that a similar tendency will be indicated even when other metal catalysts are used regarding a relative influence on the accessibility to an active site of a catalyst metal of an aromatic compound serving as a reactant and on the electron conductivity.

**[TABLE 3]**

| MASS RATIO I/C | RELATIVE CURRENT DENSITY |
|---|---|
| 0.1 | 0.82 |
| 0.3 | 1.28 |
| 0.5 | 1.35 |
| 0.8 | 1.1 |
| 1 | 1 |
| 1.5 | 0.94 |
| 2 | 0.87 |

The invention is not limited to the above-mentioned embodiments, and various modifications, such as a design change, may be added thereto on the basis of knowledge of those skilled in the art. It should be understood that any embodiment to which one or more of the modifications are added is also included in the scope of the invention.

### [DESCRIPTION OF THE REFERENCE NUMERALS]

10 electrochemical reduction device, 20 power control unit, 30 organic material storage tank, 40 water storage tank, 50 gas-liquid separation unit, 100 electrolysis cell, 110 electrolyte membrane, 120 reduction electrode, 130 oxygen evolving electrode, 140a, 140b liquid diffusion layer, 150a, 150b separator

### [INDUSTRIAL APPLICABILITY]

The present invention can be applied to technologies for electrochemically hydrogenating an aromatic hydrocarbon compound or an N-containing heterocyclic aromatic compound.

## Claims

1. An electrochemical reduction device (10) for manufacturing a hydride of an aromatic hydrocarbon compound or an N-containing heterocyclic aromatic compound by hydrogenating at least one benzene ring of said aromatic hydrocarbon compound or said N-containing heterocyclic aromatic compound comprising:
an electrolyte membrane (110) that has protonic conductivity;
a reduction electrode (120) that is provided on one side of the electrolyte membrane and that contains a reduction catalyst; and
an oxygen evolving electrode (130) that is provided on the other side of the electrolyte membrane,
wherein the reduction catalyst is an alloy or an intermetallic compound of a first catalyst metal and a second catalyst metal,
wherein said first catalyst metal is at least one chosen from the group of Pt and Pd, and said second catalyst metal is at least one selected from among Cr, Mn, Fe, Co, Ni, Cu, Zn, Mo, Ru, Sn, W, Re, Pb, and Bi,
wherein the ratio of the first catalyst metal to the total mass of the first catalyst metal and the second catalyst metal is between 10 wt% and 95 wt%, both inclusive,
wherein the first catalyst metal and the second catalyst metal are supported on a conductive material chosen from the group of a porous carbon, a porous metal, and a porous metal oxide, and
wherein the conductive material has an electrical conductivity of 1.0*10⁻² S/cm or greater.

2. The electrochemical reduction device (10) according to claim 1, wherein the reduction electrode contains a protonic conductor.

3. The electrochemical reduction device (10) according to claim 2, wherein the protonic conductor is an ion-conductive polymer.

4. The electrochemical reduction device (10) according to claim 2 or 3, wherein the reduction catalyst is partially coated by the protonic conductor.

5. A method for manufacturing a hydride of an aromatic hydrocarbon compound or an N-containing heterocyclic aromatic compound, wherein, by introducing an aromatic hydrocarbon compound or an N-containing heterocyclic aromatic compound to the reduction electrode side of the electrochemical reduction device (10) according to any one of claims 1 through 4 while circulating water or a humidified gas to the oxygen evolving electrode side and then by externally applying an electric field such that the reduction electrode (120) and the oxygen evolving electrode (130) have a less-noble potential and a noble potential, respectively, at least one benzene ring of the aromatic hydrocarbon compound or the N-containing heterocyclic aromatic compound introduced to the reduction electrode side is hydrogenated.

6. A method for manufacturing a hydride of an aromatic hydrocarbon compound or an N-containing heterocyclic aromatic compound, wherein the aromatic hydrocarbon compound or the N-containing heterocyclic aromatic compound to be introduced to the reduction electrode side is introduced in a liquid state at a reaction temperature in the method for manufacturing a hydride of an aromatic hydrocarbon compound or an N-containing heterocyclic aromatic compound according to claim 5.

## Patentansprüche

1. Elektrochemische Reduktionsvorrichtung (10) zur Herstellung eines Hydrids einer aromatischen Kohlenwasserstoffverbindung oder einer N-haltigen heterocyclischen aromatischen Verbindung durch hydrieren wenigstens eines Benzolring der aromatischen Kohlenwasserstoffverbindung oder der N-haltigen heterocyclischen aromatischen Verbindung, umfassend:
eine Elektrolytmembran (110), die eine Protonenleitfähigkeit aufweist; eine Reduktionselektrode (120), die auf einer Seite der Elektrolytmembran vorgesehen ist und die einen Reduktionskatalysator aufweist; und eine Sauerstoffentwicklungselektrode (130), die auf der anderen Seite der Elektrolytmembran vorgesehen ist, wobei der Reduktionskatalysator eine Legierung oder eine intermetallische Verbindung eines ersten Katalysatormetalls und eines zweiten Katalysatormetalls ist, wobei das erste Katalysatormetall mindestens eines ausgewählt aus der Gruppe von Pt und Pd ist und das zweite Katalysatormetall mindestens eines ausgewählt aus Cr, Mn, Fe, Co, Ni, Cu, Zn, Mo, Ru, Sn, W, Re, Pb und Bi ist, wobei das Verhältnis des ersten Katalysatormetalls zu der Gesamtmasse des ersten Katalysatormetalls und des zweiten Katalysatormetalls zwischen jeweils einschließlich 10 Gew.-% und 95 Gew.-% beträgt, wobei das erste Katalysatormetall und das zweite Katalysatormetall von einem leitfähigen Material getragen sind, das aus der Gruppe aus einem porösen Kohlenstoff, einem porösen Metall und einem porösen Metalloxid ausgewählt ist, und wobei das leitfähige Material eine elektrische Leitfähigkeit von 1,0 × 10⁻² S/cm oder mehr aufweist.

2. Elektrochemische Reduktionsvorrichtung (10) nach Anspruch 1, wobei die Reduktionselektrode einen Protonenleiter enthält.

3. Elektrochemische Reduktionsvorrichtung (10) nach Anspruch 2, wobei der Protonenleiter ein ionenleitfähiges Polymer ist.

4. Elektrochemische Reduktionsvorrichtung (10) nach Anspruch 2 oder 3, wobei der Reduktionskatalysator teilweise durch den Protonenleiter beschichtet ist.

5. Verfahren zur Herstellung eines Hydrids einer aromatischen Kohlenwasserstoffverbindung oder einer N-haltigen heterocyclischen aromatischen Verbindung, wobei durch Heranführen einer aromatischen Kohlenwasserstoffverbindung oder einer N-haltigen heterocyclischen aromatischen Verbindung an die Reduktionselektrodenseite der elektrochemischen Reduktionsvorrichtung (10) gemäß einem der Ansprüche 1 bis 4, während Wasser oder ein befeuchtetes Gas zur Seite der Sauerstoffentwicklungselektrode zirkuliert und dann durch externes Anlagen eines elektrischen Felds, so dass die Reduktionselektrode (120) und die Sauerstoffentwicklungselektrode (130) ein weniger edles Potential bzw. ein edles Potential aufweisen, mindestens ein Benzolring der an die Reduktionselektrodenseite herangeführte aromatischen Kohlenwasserstoffverbindung oder der N-haltigen heterocyclischen aromatischen Verbindung hydriert wird.

6. Verfahren zur Herstellung eines Hydrids einer aromatischen Kohlenwasserstoffverbindung oder einer N-haltigen heterocyclischen aromatischen Verbindung, wobei die aromatische Kohlenwasserstoffverbindung oder die N-haltige heterocyclische aromatische Verbindung, die an die Reduktionselektrodenseite heranzuführen ist, in einem flüssigen Zustand bei einer Reaktionstemperatur in dem Verfahren zur Herstellung eines Hydrids einer aromatischen Kohlenwasserstoffverbindung oder einer N-haltigen heterocyclischen aromatischen Verbindung nach Anspruch 5 herangeführt wird.

## Revendications

1. Dispositif de réduction électrochimique (10) pour fabriquer un hydrure d'un composé hydrocarboné aromatique ou d'un composé aromatique hétérocyclique contenant de l'azote par hydrogénation d'au moins un noyau benzénique dudit composé hydrocarboné aromatique ou dudit composé aromatique hétérocyclique contenant de l'azote comprenant :
une membrane électrolytique (110) qui a une conductivité protonique ;
une électrode réductrice (120) qui est prévue sur un côté de la membrane électrolytique et qui contient un catalyseur de réduction ; et
une électrode dégageant de l'oxygène (130) qui est prévue sur l'autre côté de la membrane électrolytique,
dans lequel le catalyseur de réduction est un alliage ou un composé intermétallique d'un premier métal catalyseur et d'un deuxième métal catalyseur,
dans lequel ledit premier métal catalyseur est au moins un métal choisi dans le groupe constitué de Pt et Pd, et ledit deuxième métal catalyseur est au moins un métal choisi parmi Cr, Mn, Fe, Co, Ni, Cu, Zn, Mo, Ru, Sn, W, Re, Pb, et Bi,
dans lequel le rapport du premier métal catalyseur sur la masse totale du premier métal catalyseur et du deuxième métal catalyseur est compris entre 10% en poids et 95% en poids, bornes incluses,
dans lequel le premier métal catalyseur et le deuxième métal catalyseur sont supportés sur un matériau conducteur choisi dans le groupe constitué d'un carbone poreux, d'un métal poreux et d'un oxyde métallique poreux, et
dans lequel le matériau conducteur a une conductivité électrique supérieure ou égale à 1,0 * 10⁻² S/cm.

2. Dispositif de réduction électrochimique (10) selon la revendication 1, dans lequel l'électrode réductrice contient un conducteur protonique.

3. Dispositif de réduction électrochimique (10) selon la revendication 2, dans lequel le conducteur protonique est un polymère conducteur d'ions.

4. Dispositif de réduction électrochimique (10) selon la revendication 2 ou 3, dans lequel le catalyseur de réduction est partiellement revêtu du conducteur protonique.

5. Procédé de fabrication d'un hydrure d'un composé hydrocarboné aromatique ou d'un composé aromatique hétérocyclique contenant de l'azote, où, par l'introduction d'un composé hydrocarboné aromatique ou d'un composé aromatique hétérocyclique contenant de l'azote au côté électrode réductrice du dispositif de réduction électrochimique (10) selon l'une quelconque des revendications 1 à 4 tout en faisant circuler de l'eau ou un gaz humidifié vers le côté électrode dégageant de l'oxygène et ensuite par l'application externe d'un champ électrique de sorte que l'électrode réductrice (120) et l'électrode dégageant de l'oxygène (130) possèdent un potentiel moins noble et un potentiel noble, respectivement, au moins un noyau benzénique du composé hydrocarboné aromatique ou du composé aromatique hétérocyclique contenant de l'azote introduit au côté électrode réductrice est hydrogéné.

6. Procédé de fabrication d'un hydrure d'un composé hydrocarboné aromatique ou d'un composé aromatique hétérocyclique contenant de l'azote, où le composé hydrocarboné aromatique ou le composé aromatique hétérocyclique contenant de l'azote à introduire au côté électrode réductrice est introduit à l'état liquide à une température de réaction dans le procédé de fabrication d'un hydrure d'un composé hydrocarboné aromatique ou d'un composé aromatique hétérocyclique contenant de l'azote selon la revendication 5.
